# EUROPEAN PATENT SPECIFICATION

(11) **EP 1 653 923 B1**
(45) Date of publication and mention of the grant of the patent: **28.04.2010**
(21) Application number: 04767956.8
(22) Date of filing: 30.07.2004
(51) Int. Cl.: A61K 9/107, A61K 9/12, A61K 9/48

(54) **ORAL DELIVERY SYSTEM COMPRISING A BILIQUID FOAM**
ORALES ABGABESYSTEM MIT EINEM BILIQUID FOAM
SYSTEME AMELIORE D'ADMINISTRATION DE MEDICAMENT

(30) Priority: 30.07.2003 GB 0317869
(43) Date of publication of application: 10.05.2006
(73) Proprietor: Drug Delivery Solutions Limited, Leatherhead, Surrey KT22 7RY (GB)
(72) Inventor: CHARMAN, William, Parkville, Victoria 3052 (AU); DIAS, Monica, Guildford Surrey GU8 4EP (GB); GEORGIOU, Michelle, Chilworth Surrey GU4 8LP (GB); GUFFOGG, Philip, Truro Cornwall TR3 7EA (GB); PORTER, Christopher, Parkville, Victoria 3052 (AU); POUTON, Colin, Parkville, Victoria 3052 (AU); STEELE, Fraser, Horsham, West Sussex RH12 5HF (GB); WHEELER, Derek, Beare Green Surrey RH5 4RJ (GB)
(74) Representative: Hayes, Adrian Chetwynd
(86) International application number: PCT/GB2004/003329
(87) International publication number: WO 2005/011628

(56) References cited:
- WO-A-01/62214
- WO-A-97/32559
- US-A- 4 486 333
- US-A- 4 999 198
- SEBBA F: "PREPARATION AND PROPERTIES OF POLYAPHRONS (BILIQUID FOAMS)" CHEMISTRY AND INDUSTRY, CHEMICAL SOCIETY. LETCHWORTH, GB, no. 10, 21 May 1984 (1984-05-21), pages 367-372, XP001149037 ISSN: 0009-3068

## Description

The present invention relates to an improved drug delivery system and, in particular, to an improved drug delivery system for the oral administration of lipophilic poorly water-soluble drugs in immediate release dosage forms.

The bioavailability of lipophilic, poorly water-soluble drugs when administered orally in solid dosage forms (such as tablets) is notoriously low and variable. This has led to the development of dosage forms in which the drug is predissolved in either a lipid vehicle or a mixture of a lipid vehicle and a surfactant or a ternary mixture of a lipid vehicle, a surfactant and a co-solvent. Such compositions provide an increased bioavailability of the drug but only at the cost of increased complexity and, in most cases, the need to include very high levels (30% or greater) of surfactant or emulsifier.

Existing lipid-based delivery vehicles for lipophilic drugs include the simple solution of the drug in a lipophilic vehicle, self-emulsifying oil systems, microemulsions and liposomes. The properties and application characteristics of lipophilic drug delivery vehicles have been the subject of numerous reviews - for example, Humberstone & Charman (1997) Advanced Drug Delivery Review v.25, 103-128 and O'Driscoll (2002) European Journal of Pharmaceutical Science v.15, 405-415.

### Lipophilic Solution.

A number of drugs have an appreciable solubility in lipophilic oils (especially triacyl glycerides) alone. It is therefore possible to administer the drug as a simple solution in a capsule and obtain satisfactory absorption and bioavailability. However, the dispersion kinetics of such a formulation cannot be expected to be as rapid as would be observed for a pre-dispersed system. The slow dispersion of the formulation is a major limitation of this dosage form.

### Self-emulsifying Oil Systems

These are sometimes referred to as SEDDS ('self-emulsifying drug delivery systems') and comprise a mixture of an oil and a surfactant that spontaneously forms an oil-in-water emulsion when diluted with water. The solubility of the drug is typically enhanced by the presence of the surfactant - which is usually present in concentrations as high as or greater than 30%. Co-solvents such as ethanol, propylene glycol and polyethylene glycol are sometimes added in order to increase the solubility of the drug. This dosage form is a lipophilic, isotropic liquid which may be filled into capsules and which, when liberated from the capsule in the gastrointestinal tract, forms a dispersion of small drug-containing oil/surfactant droplets which spread rapidly. The main disadvantage of SEDDS relates to the presence of the large amounts of surfactant, which, apart from potentially having a harmful effect on the intestinal wall, adds to the cost and complexity of the formulation. Examples of such compositions are disclosed in US Patents Nos. 6436430 and 6284268.

### Microemulsion preconcentrates

These are essentially similar to SEDDS and comprise isotropic mixtures of drug, lipid, surfactant and (if required) co-solvent and co-surfactant. As with the self-emulsifying drug delivery systems, on addition to an aqueous medium these systems disperse to form liquid/liquid dispersions. The primary difference between microemulsion preconcentrates and SEDDS is the nature of the dispersion formed, where the microemulsion preconcentrates disperse to form thermodynamically stable microemulsions.
Microemulsions have been shown to enhance the bioavailability of lipophilic drugs but suffer from the same major disadvantage as for SEDDS - the very high level of surfactant needed for their formation. Examples of such compositions are disclosed in US Patents Nos. 5993858 and 6309665.

### Liposomes

Liposomes consist of ordered layers of phospholipid molecules which encapsulate a central aqueous lumen. The possibility exists for lipophilic drugs to be solublised within the phospholipid layers. The drug carrying capabilities of liposomes are sufficient for use in parenteral formulations, but are not particularly suitable for use in oral dosage forms. Furthermore, liposomes are unstable and expensive to produce and therefore have limited potential for the delivery of lipophilic drugs. Examples of such compositions are disclosed in US Patents Nos. 4746516 and 6090407.

Other dosage forms include the conversion of microemulsions into solid or semisolid nano particles and the use of polyaphrons. US Patent No. 4999198 discloses a polyaphron comprising a continuous phase and a disperse phase in which a drug, specifically scopolamine, is carried. The patent describes the slow release of the drug from the polyaphron into a medium with which the polyaphron is in contact and in particular the transdermal delivery of drugs. The invention described here is different from that previously described in US Patent No. 4999198. No reference has previously been given to the use of such polyaphrons as an oral delivery system which is compatible with hard or soft gelatin capsules. No specific water to lipid phase ratio is given in the previous patent. Furthermore, scopolamine is the only drug specifically mentioned.

The disadvantages of the oral formulations for the delivery of lipophilic poorly water-soluble drugs have been discussed above. None of the current formulations is particularly satisfactory.

European publication no. 1,515,682 discloses a discrete powder which comprises particles in which a biliquid foam has been entrapped within a matrix of a polymeric material.

We have now developed a readily dispersible two-phase system for the oral delivery of poorly water-soluble drugs which has a low water content (less than 10% w/w water) and therefore gives the system a good compatibility with gelatin, thereby enabling the drug formulation to be encapsulated in hard or soft gelatin capsules. Furthermore, the two-phase system is simple to produce and requires the use of only a limited amount of potentially expensive and harmful surfactants.

Accordingly, the present invention provides an oral drug delivery system which comprises a biliquid foam comprising
from 1 to 20% by weight of a continuous hydrophilic phase,
from 70 to 98% by weight of a pharmaceutically acceptable oil which forms a discontinuous phase, the said pharmaceutically acceptable oil having dissolved or dispersed therein a poorly water-soluble drug in an amount of from 0.1 to 20% by weight
and the biliquid foam including therein from 0.5 to 10%, preferably 0.5 to 5%, by weight of a surfactant to enable the formation of a stable biliquid foam, all percentages being based upon the total weight of the formulation.

By the term "biliquid foam" which is used herein, which is also referred to in the art as a "polyaphron", is meant a non-isotropic dispersion of a non-polar liquid suspended in a continuous polar phase.

By the term "poorly water-soluble drug" as used herein is meant a drug which will dissolve in water in an amount of less than 1% by weight. The discontinuous phase contains the drug in an amount of 0.1 to 20% by weight, for example 1 to 10% by weight or 2 to 7% by weight. It is also possible for some drug to be present in the continuous hydrophilic phase, particularly if a cosolvent such as a polyethylene glycol is used.

The pharmaceutically acceptable oil which is used in the present invention is preferably a mono-, di- or triglyceride, or a mixture thereof. In particular the mono-, di- or triglycerides are preferably the glycerol esters of fatty acids containing from 6 to 22 carbon atoms.

Examples of oils which may be used in the present invention include almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, olive oil, palm kernel oil, palm oil, peanut oil, rapeseed oil, safflower oil, sesame oil, shark liver oil, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, modified triglycerides, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C₈-C₁₂ fatty acid chains, medium chain triglycerides, long chain triglycerides, modified triglycerides, fractionated triglycerides, and mixtures thereof.

Examples of mono and diglycerides which may be used in the present invention include propylene glycol mono and diesters having from 15 to 40 carbon atoms, including hydrolysed coconut oils (e.g. Capmul MCM), hydrolysed corn oil (e.g. Maisine 35-1).

The monoglycerides and diglycerides are mono- or di-saturated fatty acid esters of glycerol having eight to sixteen carbon chain length.

Essential oils may also be used in the present invention.

The surfactant used in the present invention may be incorporated into either or both phases of the biliquid foam. The surfactant used in the present invention is preferably an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adducts containing from 25 to 60 ethoxy groups a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof. The surfactant may be used in an amount of from 0.5 to 5% by weight of the biliquid foam but preferably is used in an amount of from 1 to 2%, by weight of the biliquid foam.

A co-emulsifier may be used in the formation of the biliquid foams in an amount sufficient to complete the solubilization of the poorly water-soluble drug. A suitable co-emulsifier is a phosphoglyceride, a phospholipid, for example lecithin, or a free fatty acid that is liquid at room temperature, for example iso-stearic acid, oleic acid, linoelic acid or linolenic acid.

The continuous hydrophilic phase of the biliquid foam may comprise water or may comprise an aqueous phase which includes therein an additional component to reduce the affinity of the aqueous phase for a capsule forming material such as gelatin. The additional component may be a salt such as sodium chloride, or a co-solvent such as an aliphatic alcohol, polyethylene glycol, propylene glycol or glycerol, or mixtures thereof, or a gelling agent such as alginate gums or their salts, guar gum, locust bean gum, xanthan gum, gum acacia, gelatin, hydroxymethyl-cellulose hydroxyethylcellulose, hydroxypropyl-cellulose, carboxymethylcellulose or its salts, bentonites, magnesium aluminium silicates, "Carbomers" (salts of cross-linked polymers of acrylic acid), or glyceryl polymethacrylates or their dispersions in glycols, or a polyvinylpyrrolidone polymer or a water-dispersible copolymer thereof, or any appropriate mixture of any of these polymers and gums.

Alternatively, the hydrophilic phase may be non-aqueous and may be, for example, an aliphatic alcohol, polyethylene glycol, propylene glycol or glycerol, or mixtures thereof.

Water-soluble inorganic salts may be added to improve the stability of the biliquid foams, such as those formed from monovalent cations such as Na⁺, K⁺ or NH₄⁺, divalent cations such as Ca⁺⁺ or Mg⁺⁺ or trivalent cations such as Al⁺⁺⁺. Water soluble polysaccharides such as sucrose, glucose or fructose may also be added to improve stability.

Poorly water-soluble drugs which may be used in the present invention include the following:
Analgesics and anti-inflammatory agents: aloxiprin, auranofin, azapropazone, benorylate, diflunisal, etodolac, fenbufen, fenoprofen calcium, flurbiprofen, ibuprofen, indomethacin, ketoprofen, mefenamic acid, nabumetone, naproxen, oxyphenbutazone, phenylbutazone, piroxicam, sulindac.
Anthelmintics: albendazole, bephenium hydroxynaphthoate, dichlorophen, ivermectin, mebendazole, oxfendazole, oxantel embonate, praziquantel, pyrantel embonate, thiabendazole.
Anti-arrhythmic agents: amiodarone HCl, disopyramide, quinidine sulphate.
Anti-bacterial agents: benethamine penicillin, cinoxacin, ciprofloxacin HCl, clarithromycin, clofazimine, cloxacillin, doxycycline, erythromycin, ethionamide, imipenem, nalidixic acid, nitrofurantoin, rifampicin, spiramycin, sulphabenzamide, sulphadoxine, sulphamerazine, sulphacetamide, sulphadiazine, sulphafurazole, sulphamethoxazole, sulphapyridine, tetracycline, trimethoprim.
Anti-coagulants: dicoumarol, dipyridamole, nicoumalone, phenindione.
Anti-depressants: amoxapine, maprotiline HCl, trimipramine maleate.
Anti-diabetics: acetohexamide, chlorpropamide, glibenclamide, gliclazide, glipizide, tolazamide, tolbutamide.
Anti-epileptics: beclamide, carbamazepine, clonazepam, ethotoin, methoin, methsuximide, methylphenobarbitone, phenacemide, phenobarbitone, phenytoin, phensuximide, primidone, sulthiame, valproic acid.
Anti-fungal agents: amphotericin, butoconazole nitrate, clotrimazole, econazole nitrate, fluconazole, griseofulvin, itraconazole, ketoconazole, miconazole, natamycin, nystatin, sulconazole nitrate, terbinafine HCl, terconazole, tioconazole, undecenoic acid.
Anti-gout agents: allopurinol, probenecid, sulphin-pyrazone.
Anti-hypertensive agents: amlodipine, diazoxide, felodipine, isradipine, minoxidil, nicardipine HCl, nifedipine, nimodipine, prazosin HCl, reserpine.
Anti-malarials: amodiaquine, chloroquine, halofantrine HCl, mefloquine HCl, proguanil HCl, pyrimethamine, quinine sulphate.
Anti-migraine agents: dihydroergotamine mesylate, ergotamine tartrate, methysergide maleate, pizotifen maleate.
Anti-muscarinic agents: atropine, benzhexol HCl, biperiden, hyoscyamine, mepenzolate bromide, tropicamide.
Anti-neoplastic agents and Immunosuppressants: aminoglutethimide, azathioprine, busulphan, chlorambucil, cyclosporin, dacarbazine, etoposide, lomustine, melphalan, mercaptopurine, methotrexate, mitomycin, mitotane, tamoxifen citrate, testolactone.
Anti-protazoal agents: clioquinol, diiodohydroxyquinoline, diloxanide furoate, dinitolmide, furzolidone, metronidazole, nitrofurazone, tinidazole.
Anti-thyroid agents: carbimazole, propylthiouracil.
Anxiolytic, sedatives, hypnotics and neuroleptics: alprazolam, amylobarbitone, barbitone, bromazepam, bromperidol, brotizolam, butobarbitone, carbromal, chlordiazepoxide, chlormethiazole, chlorpromazine, clobazam, clozapine, diazepam, droperidol, ethinamate, fluanisone, flunitrazepam, fluopromazine, flupenthixol decanoate, fluphenazine decanoate, flurazepam, haloperidol, lorazepam, lormetazepam, medazepam, meprobamate, methaqualone, midazolam, nitrazepam, oxazepam, pentobarbitone, perphenazine pimozide, prochlorperazine, sulpiride, temazepam, thioridazine, triazolam, zopiclone.
β-Blockers: nadolol, pindolol.
Cardiac Inotropic agents: digitoxin, digoxin, lanatoside C, medigoxin.
Corticosteroids: beclomethasone, betamethasone, budesonide, cortisone acetate, desoxymethasone, dexamethasone, fludrocortisone acetate, flunisolide, flucortolone, fluticasone propionate, hydrocortisone, methylprednisolone, prednisolone, prednisone, triamcinolone.
Diuretics: acetazolamide, amiloride, bendrofluazide, bumetanide, chlorothiazide, chlorthalidone, ethacrynic acid, frusemide, metolazone, spironolactone, triamterene.
Anti-parkinsonian agents: bromocriptine mesylate.
Gastro-intestinal agents: bisacodyl, cimetidine, cisapride, diphenoxylate HCl, domperidone, famotidine, loperamide, mesalazine, omeprazole, sulphasalazine.
Histamine H,-Receptor Antagonists: astemizole, cinnarizine, cyclizine, cyproheptadine HCl, dimenhydrinate, meclozine HCl, oxatomide, terfenadine.
Lipid regulating agents: bezafibrate, clofibrate, fenofibrate, gemfibrozil, probucol.
Nitrates and other anti-anginal agents: amyl nitrate, glyceryl trinitrate, isosorbide dinitrate, pentaerythritol tetranitrate.
Nutritional agents: betacarotene, vitamin A, vitamin B2, vitamin D, vitamin E, vitamin K.
Opioid analgesics: codeine, dextropropyoxyphene, diamorphine, dihydrocodeine, meptazinol, morphine, pentazocine.
Sex hormones: clomiphene citrate, danazol, ethinyl estradiol, medroxyprogesterone acetate, mestranol, methyltestosterone, norethisterone, norgestrel, estradiol, conjugated oestrogens, progesterone, stanozolol, stibestrol, testosterone, tibolone.
Stimulants: dexamphetamine, dexfenfluramine, mazindol.

Pharmaceutically acceptable salts, isomers and derivatives thereof may be substituted for these drugs. Mixtures of lipophilic drugs may be used where therapeutically effective.

The discontinuous phase of the present invention comprises 70 to 98% by weight, preferably from 80 to 96% by weight, more preferably from 90 to 95% by weight of the biliquid foam. The continuous hydrophilic phase comprises from 1 to 20% by weight, preferably from 2 to 10% by weight of the biliquid foam.

The oral drug delivery systems of the present invention are preferably presented in a unit dosage form. The preferred unit dosage form comprises capsules filled with the biliquid foam, for example hard or soft gelatin capsules. The use of the gelatin capsules is made possible by the low water content of the biliquid foam which ensures good compatibility both with the hard and soft gelatin capsules and the optional incorporation into the aqueous phase of an additional component which reduces the affinity of the aqueous phase for the capsule material. This is an advantage over the currently available lipid dispersions and provides a better bioavailability of the drug as compared to tablets.

Each unit dosage form will comprise, for example, from 0.5mg to 1000mg, preferably 0.5 to 200mg of the drug, for example in up to a 1000mg, preferably 100mg, dosage form.

The biliquid foams of the drug delivery systems may also be presented as dilutable concentrates which are infinitely dilutable in a co-solvent such as water or a water compatible aliphatic alcohol, polyethylene glycol, propylene glycol or glycerol, or mixtures thereof. Dilution of the biliquid foam preparations is possible and they may be incorporated into a drink, syrup or linctus.

The biliquid foam compositions of the present invention may also contain other additives such as preservatives or antimicrobial agents(for instance to prevent microbiological spoilage). These additives may be included in the non-polar liquid or the continuous phase.

It will be understood that the inclusion of these additives will be at the levels and with the type of materials which are found to be effective and useful. Care needs to be taken in the choice and amount of these additives to prevent compromise to the other performance advantages of the present invention.

Methods of producing biliquid foams are described in US-A-4486333 involving the preliminary formation of a gas foam in order to provide a sufficiently large surface area on which the biliquid foam can subsequently be formed. It has been found that the prior formation of a gas foam is not required to manufacture a stable biliquid foam, provided that a suitable stirring mechanism is provided in the manufacturing vessel.

Such an apparatus comprises a tank provided with a stirrer in which the stirrer blade breaks the interface between the liquid and air. A delivery device is provided through which the oil phase (non-polar liquid), which will comprise the internal phase of the dispersion is delivered to the tank. The design of the delivery device is such that the rate of addition of the internal phase fluid can be controlled and varied during the production process. A feature of the production process is that the internal (oil) phase is added to the stirred aqueous phase slowly at first until sufficient droplets have been formed to constitute a large surface area for the more rapid formation of new droplets. At this point, the rate of addition of the oil phase may be increased.

The production process consists of the following steps:
1. The addition of one or more chosen surfactants to one or other or both phases (as previously determined by experiment).
2. The charging of the aqueous phase into the bottom of a process vessel.
3. The incorporation of the stirrer into the vessel so that it stirs the surface of the aqueous phase.
4. Adjustment of the stirrer speed to a previously determined level.
5. The slow addition of the internal (oil) phase containing the poorly water-soluble drug dissolved or dispersed therein whilst continuing to stir at the prescribed speed.
6. The speeding up of the rate of addition of the oil phase once a prescribed amount (usually between 5% and 10% of the total amount to be added) has been added.

The stirring rate and the rate of addition of the oil phase are variables, the values of which depend upon the detailed design of the manufacturing plant (in particular, the ratio of tank diameter to impeller diameter), the physico-chemical properties of the oil phase and the nature and concentrations of the chosen surfactants. These can all be pre-determined by laboratory or pilot plant experiment.

It will be understood by those skilled in the art that other manufacturing methods may be used, as appropriate.

Although the stability of the biliquid foams is generally good, they may be stabilised by the addition of an aqueous gel and, accordingly, the present invention includes within its scope a stable dispersion which comprises from 1 to 80% by weight of a biliquid foam and from 20 to 99% by weight of an aqueous gel.

The aqueous gel will preferably be formed from a colloidal polymer or gum suspended in water, at a concentration of from 0.05 to 20% by weight, more preferably from 0.2 to 1% by weight. Suitable polymers or gums are, for example, alginate gums or their salts, guar gum, locust bean gum, xanthan gum, gum acacia, gelatin, hydroxymethylcellulose hydroxyethylcellulose, hydroxypropylcellulose, carboxymethylcellulose or its salts, bentonites, magnesium aluminium silicates, "Carbomers" (salts of cross-linked polymers of acrylic acid), or glyceryl polymethacrylates or their dispersions in glycols, or any appropriate mixture of any of these polymers and gums.

The present invention will be further described with reference to the following examples:

### Biliquid Foam Preparation

A suitable vessel was charged with the aqueous phase of the biliquid foam. The drug was dissolved in the oil phase. The oil phase containing the drug was then added at a constant rate with stirring, using a sweep stirrer or an orbital mixer. After completion of the oil addition, the stirring was continued until the size of the oil droplets became stable or reached a desired size.

### Example 1

| **Oil phase** | **%** | **Weight(g)** |
|---|---|---|
| Caprylic/capric triglyceride | 90 | 27 |
| Halofantrine | 5 | 1.5 |
| | | |

| **Aqueous phase** | | |
|---|---|---|
| Castor oil/polyoxyethylene | 1 | 0.3 |
| glycol (35) adduct | | |
| Deionised water | 4 | 1.2 |
| Total | 100 | 30.0 |

### Example 2

| **Oil phase** | **%** | **Weight (g)** |
|---|---|---|
| Caprylic/capric triglyceride | 90 | 27 |
| Halofantrine | 5 | 1.5 |
| | | |

| **Aqueous phase** | | |
|---|---|---|
| Hydrogenated | 1 | 0.3 |
| castor oil/polyoxyethylene | | |
| glycol (40) adduct | | |
| Deionised water | 4 | 1.2 |
| Total | 100 | 30.0 |

### Example 3

| **Oil phase** | **%** | **Weight(g)** |
|---|---|---|
| Caprylic/capric triglyceride | 90 | 27 |
| Halofantrine | 5 | 1.5 |
| | | |

| **Aqueous phase** | | |
|---|---|---|
| Hydrogenated | 1 | 0.3 |
| castor oil/polyoxyethylene | | |
| glycol (60) adduct | | |
| Deionised water | 4 | 1.2 |
| Total | 100 | 30.0 |

### Example 4

| **Oil phase** | **%** | **Weight(g)** |
|---|---|---|
| Soybean oil BP | 90 | 27 |
| Halofantrine | 5 | 1.5 |
| | | |

| **Aqueous phase** | | |
|---|---|---|
| Hydrogenated | 1 | 0.3 |
| castor oil/polyoxyethylene | | |
| glycol (35) adduct | | |
| Deionised water | 4 | 1.2 |
| Total | 100 | 30.0 |

### Example 5

| **Oil Phase** | **%** | **Weight (g)** |
|---|---|---|
| Caprylic/capric | 90 | 27 |
| triglycerides | | |
| Cyclosporin | 5 | 1.5 |
| | | |

| **Aqueous Phase** | | |
|---|---|---|
| Hydrogenated castor oil/ | 1 | 0.3 |
| polyoxyethylene | | |
| glycol (60) adduct | | |
| Deionised water | 4 | 1.2 |
| Total | 100 | 30.0 |

### Example 6

| **Oil Phase** | **%** | **Weight (g)** |
|---|---|---|
| Caprylic/capric triglycerides | 40 | 12 |
| Glyceryl monolinoleate | 40 | 12 |
| (Maisine 35) | | |
| Cyclosporin | 10 | 3.0 |
| | | |

| **Aqueous Phase** | | |
|---|---|---|
| Hydrogenated castor oil/ | 1 | 0.3 |
| polyoxyethylene glycol | | |
| (60) adduct | | |
| 1% aqueous calcium | 9 | 2.7 |
| chloride solution | | |
| Total | 100 | 30..0 |

### Example 7

| **Oil Phase** | **%** | **Weight (g)** |
|---|---|---|
| Soybean Oil BP | 85.5 | 25.65 |
| Halofantrine | 4.5 | 1.35 |
| | | |

| **Aqueous Phase** | | |
|---|---|---|
| Castor oil/polyoxyethylene | 2 | 0.6 |
| glycol (60) adduct | | |
| Sodium chloride | 1 | 0.3 |
| Deionised water | 7 | 2.1 |
| Total | 100 | 30.0 |

### Example 8

The following formulation could be prepared:

| **Oil Phase** | **%** | **Weight (g)** |
|---|---|---|
| Soybean Oil BP | 75.2 | 22.56 |
| Halofantrine | 4 | 1.2 |
| Oleic Acid | 0.8 | 0.24 |
| | | |

| **Aqueous Phase** | | |
|---|---|---|
| Ethanol (DEB 100) | 14 | 4.2 |
| Deionised water | 5.6 | 1.68 |
| Hydrogenated castor oil/ | 0.4 | 0.12 |
| polyoxyethylene | | |
| glycol (45) adduct | | |
| Total | 100 | 30.0 |

Examples 9, 10, 11 and 12 show formulations containing high concentrations of propylene glycol as a co-solvent for poorly water-soluble drugs.

### Example 9

The following formulation could be prepared:

| **Oil Phase** | **%** | **Weight (g)** |
|---|---|---|
| Caprylic/capric triglycerides | 85 | 25.5 |
| Halofantrine | 4 | 1.2 |
| Hydrogenated castor oil/ | 1 | 0.3 |
| polyoxyethylene | | |
| glycol (40) adduct | | |
| | | |

| **Aqueous Phase** | | |
|---|---|---|
| Propylene glycol | 9.5 | 2.85 |
| Deionised water | 0.5 | 0.15 |
| Total | 100 | 30.0 |

### Example 10

The following formulation could be prepared:

| **Oil Phase** | **%** | **Weight (g)** |
|---|---|---|
| Caprylic/capric triglycerides | 85 | 25.5 |
| Halofantrine | 4 | 1.2 |
| Castor oil/polyoxyethylene | 1 | 0.3 |
| glycol (35) adduct | | |
| | | |

| **Aqueous Phase** | | |
|---|---|---|
| Propylene glycol | 9.5 | 2.85 |
| Deionised water | 0.5 | 0.15 |
| Total | 100 | 30.0 |

### Example 11

The following formulation could be prepared:

| **Oil Phase** | **%** | **Weight (g)** |
|---|---|---|
| Soybean Oil BP | 84 | 25.2 |
| Halofantrine | 5 | 1.5 |
| Castor oil/polyoxyethylene | 1 | 0.3 |
| glycol (35) adduct | | |
| | | |

| **Aqueous Phase** | | |
|---|---|---|
| Propylene glycol | 9 | 2.7 |
| Deionised water | 1 | 0.3 |
| Total | 100 | 30.0 |

### Example 12

The following formulation could be prepared:

| **Oil Phase** | **%** | **Weight (g)** |
|---|---|---|
| Soybean Oil BP | 84 | 25.2 |
| Halofantrine | 5 | 1.5 |
| Castor oil/polyoxyethylene | 1 | 0.3 |
| glycol (40) adduct | | |
| | | |

| **Aqueous Phase** | | |
|---|---|---|
| Propylene glycol | 9.5 | 2.85 |
| Deionised water | 0.5 | 0.15 |
| Total | 100 | 30.0 |

Example 13 illustrates the use of glycerine as a co-solvent (for poorly water-soluble drugs) in the continuous phase.

### Example 13

The following formulation could be prepared:

| **Oil Phase** | **%** | **Weight (g)** |
|---|---|---|
| Caprylic/capric triglycerides | 84 | 25.2 |
| Halofantrine | 5 | 1.5 |
| C12-13 Pareth-3 | 1 | 0.3 |
| | | |

| **Aqueous Phase** | | |
|---|---|---|
| Glycerine BP | 7 | 2.1 |
| 1% aqueous sodium laureth | 3 | 0.9 |
| sulphate | | |
| Total | 100 | 30.0 |

Examples 14 and 15 illustrate the use of polyethyleneglycols as co-solvents for poorly water-soluble drugs.

### Example 14

The following formulation could be prepared:

| **Oil Phase** | **%** | **Weight (g)** |
|---|---|---|
| Caprylic/capric triglycerides | 84 | 25.2 |
| Halofantrine | 5 | 1.5 |
| C12-13 Pareth-3 | 1 | 0.3 |
| | | |

| **Aqueous Phase** | | |
|---|---|---|
| PEG-6 | 5 | 1.5 |
| 1% aqueous sodium laureth | 5 | 1.5 |
| sulphate | | |
| Total | 100 | 30.0 |

### Example 15

The following formulation could be prepared:

| **Oil Phase** | **%** | **Weight (g)** |
|---|---|---|
| Soybean Oil BP | 84 | 25.2 |
| Halofantrine | 5 | 1.5 |
| Castor oil/polyoxyethylene | 1 | 0.3 |
| glycol (40) adduct | | |
| | | |

| **Aqueous Phase** | | |
|---|---|---|
| PEG-6 | 10 | 3 |
| Total | 100 | 30.0 |

### Example 16

In order to demonstrate the advantages of the present invention a test was carried out to compare formulations of the present invention with a tablet.

A commercial formulation Halfan^{®} (Batch no. 558, SmithKline & French, UK) was tested. Analysis showed that it contained 248 mg Halofantrine. The bioavailability was tested in fasted male beagle dogs and compared with that obtained using the formulation of Example 7 (LCT BLF) and the formulation of Example 7 except that the soybean oil is replaced with caprylic/capric triglycerides (MCT BLF). The dogs, weighing from 12 to 19 kg, were dosed in a randomised crossover study. The dogs were fasted for 21 hours prior to dosing. Blood samples were collected at -15 min (pre-close blank) and subsequently at 15, 30, 60 and 90 mins and at 2, 3, 4, 6, 8, 10, 24, 32, 48 and 72 hours post-dosing. The following results were obtained:

| **Parameter** | **Tablet** | **MCT BLF** | **LCT BLF** |
|---|---|---|---|
| Cₘₐₓ (ng/ml) | 85 | 176 | 781 |
| tₘₐₓ (h) | 1.3 | 3.8 | 2.3 |
| AUC^{0-∞} (ng.ml/h) | 1131 | 2800 | 7754 |
| Relative bioavailability compared with tablet (%) | 100 | 248 | 686 |

| | | | |
|---|---|---|---|
| Cₘₐₓ = concentration maximum measured in blood after oral administration. Tₘₐₓ = time from administration taken to reach Cₘₐₓ. AUC = Area under curve: a measure of the total amount appearing in the blood over time. | | | |

Relative bioavailability compared with tablets(%) = Relative bioavailability compared to that from the tablet, expressed as a percentage.

## Claims (Claims for the following Contracting State(s): DE, FR, GB)

1. An oral drug delivery system which comprises a biliquid foam comprising:
from 1 to 20% by weight of a continuous hydrophilic phase,
from 70 to 98% by weight of a pharmaceutically acceptable oil which forms a discontinuous phase, the said pharmaceutically acceptable oil having dissolved or dispersed therein a poorly water-soluble drug in an amount of from 0.1 to 20% by weight,
wherein the poorly water-soluble drug is a drug which will dissolve in water in an amount of less than 1% by weight,
and the biliquid foam including therein from 0.5 to 5% by weight of a surfactant to enable the formation of a stable biliquid foam, all percentages being based upon the total weight of the formulation.

2. An oral drug delivery system as claimed in claim 1 wherein the continuous hydrophilic phase is an aqueous phase.

3. An oral drug delivery system as claimed in claim 2 wherein the aqueous phase is water.

4. An oral drug delivery system as claimed in claim 2 wherein the aqueous phase incorporates a salt or a co-solvent therein.

5. An oral drug delivery system as claimed in claim 1 wherein the continuous hydrophilic phase is a non-aqueous solvent.

6. An oral drug delivery system as claimed in claim 5 wherein the non-aqueous solvent is an aliphatic alcohol, polyethylene glycol, propylene glycol or glycerol, or mixtures thereof.

7. An oral drug delivery system as claimed in any one of the preceding claims wherein the pharmaceutically acceptable oil is a mono-, di-or triglyceride, or a mixture thereof.

8. An oral drug delivery system as claimed in claim 7 wherein the mono-, di-or triglycerides are the glycerol esters of fatty acids containing from 6 to 22 carbon atoms.

9. An oral drug delivery system as claimed in any one of the preceding claims wherein the surfactant comprises an alkyl polyglycol ether, an alkyl polyglycol ester, an ethoxylated alcohol, a polyoxyethylene sorbitan fatty acid ester, a polyoxyethylene fatty acid ester, a polyoxyethylene fatty acid ester, an ionic or non-ionic surfactant, a hydrogenated castor oil/polyoxyethylene glycol adduct containing from 25 to 60 ethoxy groups, a castor oil/polyoxyethylene glycol adduct containing from 25 to 45 ethoxy groups, or mixtures thereof.

10. An oral drug delivery system as claimed in any one of the preceding claims which includes therein a co-emulsifier in an amount sufficient to complete the solubilization of the poorly water-soluble drug.

11. An oral drug delivery system as claimed in claim 10 wherein the co-emulsifier is a phosphoglyceride or a phospholipid.

12. An oral drug delivery system as claimed in any one of the preceding claims wherein the discontinuous phase comprises from 85 to 96% by weight of the biliquid foam.

13. An oral drug delivery system as claimed in claim 12 wherein the discontinuous phase comprises from 90 to 95% by weight of the biliquid foam.

14. An oral drug delivery system as claimed in any one of the preceding claims wherein the continuous hydrophilic phase comprises from 2 to 10% by weight of the biliquid foam.

15. An oral drug delivery system as claimed in any one of the preceding claims wherein the poorly water-soluble drug is an analgesic or anti-inflammatory agent, an anthelmintic, an anti-arrhythmic agent, an anti-coagulant, an antidepressant, an anti-diabetic, an anti-epileptic, an anti-fungal agent, an anti-gout agent, an anti-hypertension agent, an anti-malarial, an anti-migraine agent, an anti-muscarinic agent, an anti-neoplastic agent, an anti-protozoal agent, an anti-thyroid agent, an anxiolytic, sedative, hypnotic or neuroleptic agent, a corticosteroid, a diuretic, an anti-Parkinsonian agent, a gastro-intestinal agent, a histamine H-receptor antagonist, a lipid regulating agent, an anti-anginal agent, a nutritional agent, an opiod analgesic, a sex hormone, a stimulant, or a therapeutic mixture thereof.

16. An oral drug delivery system as claimed in any one of the preceding claims which is in a unit dosage form.

17. An oral drug delivery system as claimed in claim 16 wherein the unit dosage form comprises capsules filled with the biliquid foam.

18. An oral drug delivery system as claimed in claim 17 wherein the capsules are hard or soft gelatin capsules.

19. An oral drug delivery system as claimed in any one of claims 1 to 15 which is in the form of a dilutable concentrate.

20. An oral drug delivery system as claimed in claim 19 which is infinitely dilutable in a co-solvent.

21. An oral drug delivery system as claimed in any one of the preceding claims for use in a method of treatment by oral administration to the human or animal body.

## Claims (Claims for the following Contracting State(s): DE, FR, GB)

1. An oral drug delivery system which comprises a biliquid foam comprising:
from 1 to 20% by weight of a continuous hydrophilic phase,
from 70 to 98% by weight of a pharmaceutically acceptable oil which forms a discontinuous phase, the said pharmaceutically acceptable oil having dissolved or dispersed therein a poorly water-soluble drug in an amount of from 0.1 to 20% by weight,
wherein the poorly water-soluble drug is a drug which will dissolve in water in an amount of less than 1% by weight,
and the biliquid foam including therein from 0.5 to 5% by weight of a surfactant to enable the formation of a stable biliquid foam, all percentages being based upon the total weight of the formulation,
with the proviso that the oral drug delivery system does not comprise a biliquid foam containing:
**Aqueous Phase**
| | |
|---|---|
| Water | 9.9% |
| Tween 20 (Polysorbate 20) | 0.1% |
**Oil Phase**
| | |
|---|---|
| Ibuprofen | 4.5% |
| Isopropyl myristate | 84.5% |
| Laureth 3 | 1.0%. |

2. An oral drug delivery system as claimed in claim 1 wherein the continuous hydrophilic phase is an aqueous phase.

3. An oral drug delivery system as claimed in claim 2 wherein the aqueous phase is water.

4. An oral drug delivery system as claimed in claim 2 wherein the aqueous phase incorporates a salt or a co-solvent therein.

5. An oral drug delivery system as claimed in claim 1 wherein the continuous hydrophilic phase is a non-aqueous solvent.

6. An oral drug delivery system as claimed in claim 5 wherein the non-aqueous solvent is an aliphatic alcohol, polyethylene glycol, propylene glycol or glycerol, or mixtures thereof.

7. An oral drug delivery system as claimed in any one of the preceding claims wherein the pharmaceutically acceptable oil is a mono-, di-or triglyceride, or a mixture thereof.

8. An oral drug delivery system as claimed in claim 7 wherein the mono-, di-or triglycerides are the glycerol esters of fatty acids containing from 6 to 22 carbon atoms.

9. An oral drug delivery system as claimed in any one of the preceding claims wherein the surfactant comprises an alkyl polyglycol ether, an alkyl polyglycol ester, an ethoxylated alcohol, a polyoxyethylene sorbitan fatty acid ester, a polyoxyethylene fatty acid ester, a polyoxyethylene fatty acid ester, an ionic or non-ionic surfactant, a hydrogenated castor oil/polyoxyethylene glycol adduct containing from 25 to 60 ethoxy groups, a castor oil/polyoxyethylene glycol adduct containing from 25 to 45 ethoxy groups, or mixtures thereof.

10. An oral drug delivery system as claimed in any one of the preceding claims which includes therein a co-emulsifier in an amount sufficient to complete the solubilization of the poorly water-soluble drug.

11. An oral drug delivery system as claimed in claim 10 wherein the co-emulsifier is a phosphoglyceride or a phospholipid.

12. An oral drug delivery system as claimed in any one of the preceding claims wherein the discontinuous phase comprises from 85 to 96% by weight of the biliquid foam.

13. An oral drug delivery system as claimed in claim 12 wherein the discontinuous phase comprises from 90 to 95% by weight of the biliquid foam.

14. An oral drug delivery system as claimed in any one of the preceding claims wherein the continuous hydrophilic phase comprises from 2 to 10% by weight of the biliquid foam.

15. An oral drug delivery system as claimed in any one of the preceding claims wherein the poorly water-soluble drug is an analgesic or anti-inflammatory agent, an anthelmintic, an anti-arrhythmic agent, an anti-coagulant, an antidepressant, an anti-diabetic, an anti-epileptic, an anti-fungal agent, an anti-gout agent, an anti-hypertension agent, an anti-malarial, an anti-migraine agent, an anti-muscarinic agent, an anti-neoplastic agent, an anti-protozoal agent, an anti-thyroid agent, an anxiolytic, sedative, hypnotic or neuroleptic agent, a corticosteroid, a diuretic, an anti-Parkinsonian agent, a gastro-intestinal agent, a histamine H-receptor antagonist, a lipid regulating agent, an anti-anginal agent, a nutritional agent, an opiod analgesic, a sex hormone, a stimulant, or a therapeutic mixture thereof.

16. An oral drug delivery system as claimed in claim 15 wherein the poorly water-soluble drug is an anthelmintic, an anti-arrhythmic agent, an anti-coagulant, an antidepressant, an anti-diabetic, an anti-epileptic, an anti-fungal agent, an anti-gout agent, an anti-hypertension agent, an anti-malarial, an anti-migraine agent, an anti-muscarinic agent, an anti-neoplastic agent, an anti-protozoal agent, an anti-thyroid agent, an anxiolytic, sedative, hypnotic or neuroleptic agent, a corticosteroid, a diuretic, an anti-Parkinsonian agent, a gastro-intestinal agent, a histamine H-receptor antagonist, a lipid regulating agent, an anti-anginal agent, a nutritional agent, an opiod analgesic, a sex hormone, a stimulant, or a therapeutic mixture thereof.

17. An oral drug delivery system as claimed in any one of the preceding claims which is in a unit dosage form.

18. An oral drug delivery system as claimed in claim 17 wherein the unit dosage form comprises capsules filled with the biliquid foam.

19. An oral drug delivery system as claimed in claim 18 wherein the capsules are hard or soft gelatin capsules.

20. An oral drug delivery system as claimed in any one of claims 1 to 16 which is in the form of a dilutable concentrate.

21. An oral drug delivery system as claimed in claim 20 which is infinitely dilutable in a co-solvent.

22. An oral drug delivery system as claimed in any one of the preceding claims for use in a method of treatment by oral administration to the human or animal body.

## Patentansprüche (Patentansprüche für folgende(n) Vertragsstaat(en): DE, GB, FR)

1. Orales Wirkstofftransportsystem, das einen biliquiden Schaum umfasst, umfassend:
1 bis 20 Gewichts-% einer kontinuierlichen hydrophilen Phase,
70 bis 98 Gewichts-% eines pharmazeutisch akzeptablen Öls, das eine diskontinuierliche Phase bildet, wobei das pharmazeutisch akzeptable Öl einen schlecht wasserlöslichen Wirkstoff in einer Menge von 0,1 bis 20 Gewichts-% darin aufgelöst oder dispergiert hat,
wobei der schlecht wasserlösliche Wirkstoff ein Wirkstoff ist, der sich in Wasser in einer Menge von weniger als 1 Gewichts-% auflöst,
und der biliquide Schaum 0,5 bis 5 Gewichts-% eines Tensids darin umfasst, um die Bildung eines stabilen biliquiden Schaums zu ermöglichen, wobei alle Prozentangaben auf das Gesamtgewicht der Formulierung bezogen sind, mit der Maßgabe, dass das orale Wirkstofftransportsystem keinen biliquiden Schaum umfasst, der:
**wässrige Phase**
| | |
|---|---|
| Wasser | 9,9% |
| Tween 20 (Polysorbat 20) | 0,1% |
**Ölphase**
| | |
|---|---|
| Ibuprofen | 4,5% |
| Isopropylmyristat | 84,5% |
| Laureth-3 | 1 % |
enthält.

2. Orales Wirkstofftransportsystem nach Anspruch 1, wobei die kontinuierliche hydrophile Phase eine wässrige Phase ist.

3. Orales Wirkstofftransportsystem nach Anspruch 2, wobei die wässrige Phase Wasser ist.

4. Orales Wirkstofftransportsystem nach Anspruch 2, wobei die wässrige Phase ein Salz oder ein Kolösungsmittel darin umfasst.

5. Orales Wirkstofftransportsystem nach Anspruch 1, wobei die kontinuierliche hydrophile Phase ein nicht-wässriges Lösungsmittel ist.

6. Orales Wirkstofftransportsystem nach Anspruch 5, wobei das nicht-wässrige Lösungsmittel ein aliphatischer Alkohol, Polyethylenglycol, Propylenglycol oder Glycerol oder Mischungen davon ist.

7. Orales Wirkstofftransportsystem nach einem der vorherigen Ansprüche, wobei das pharmazeutisch akzeptable Öl ein Mono-, Di- oder Triglycerid oder eine Mischung davon ist.

8. Orales Wirkstofftransportsystem nach Anspruch 7, wobei die Mono-, Di- oder Triglyceride die Glycerolester von Fettsäuren sind, die 6 bis 22 Kohlenstoffatome enthalten.

9. Orales Wirkstofftransportsystem nach einem der vorherigen Ansprüche, wobei das Tensid einen Alkylpolyglycolether, einen Alkylpolyglycolester, einen ethoxylierten Alkohol, einen Polyoxyethylensorbitanfettsäureester, einen Polyoxyethylenfettsäureester, einen Polyoxyethylenfettsäureester, ein ionisches oder nicht-ionisches Tensid, ein hydriertes Castoröl/Polyoxyethylenglycoladdukt, das 25 bis 60 Ethoxygruppen enthält, ein Castoröl/Polyoxyethylenglycoladdukt, das 25 bis 45 Ethoxygruppen enthält oder Mischungen davon umfasst.

10. Orales Wirkstofftransportsystem nach einem der vorherigen Ansprüche, das darin einen Co-Emulgator in einer Menge umfasst, die ausreichend ist, um das Löslichmachen des schlecht wasserlöslichen Wirkstoffs zu vollenden.

11. Orales Wirkstofftransportsystem nach Anspruch 10, wobei der Co-Emulgator ein Phosphoglycerid oder ein Phospholipid ist.

12. Orales Wirkstofftransportsystem nach einem der vorherigen Ansprüche, wobei die diskontinuierliche Phase 85 bis 96 Gewichts-% des biliquiden Schaums umfasst.

13. Orales Wirkstofftransportsystem nach Anspruch 12, wobei die diskontinuierliche Phase 90 bis 95 Gewichts-% des biliquiden Schaums umfasst.

14. Orales Wirkstofftransportsystem nach einem der vorherigen Ansprüche, wobei die kontinuierliche hydrophile Phase 2 bis 10 Gewichts-% des biliquiden Schaums umfasst.

15. Orales Wirkstofftransportsystem nach einem der vorherigen Ansprüche, wobei der schlecht wasserlösliche Wirkstoff ein analgetisches oder anti-inflammatorisches Mittel ist.

16. Orales Wirkstofftransportsystem nach Anspruch 15, wobei der schlecht wasserlösliche Wirkstoff ein Anthelmintikum, ein anti-arrhythmisches Mittel, ein anti-Koagulanz, ein anti-Deppressivum, ein anti-Diabetikum, ein anti-Epileptikum, ein anti-Pilzmittel, ein anti-Gichtmittel, ein anti-Hypertoniemittel, ein anti-Malaria-, ein anti-Migränemittel, ein anti-Muskarinikum, ein anti-Neoplastikum, ein anti-Protozoenmittel, ein anti-Schilddrüsenmittel, ein Anxiolytikum, Sedativum, hypnotisches oder neuroleptisches Mittel, ein Corticosteroid, in Diuretikum, ein anti-Parkinsonmittel, ein Mittel für den Magen-Darm-Trakt, ein Histamin-H Rezeptor Antagonist, ein Lipid-Regulierungsmittel, ein anti-anginöses Mittel, ein Ernährungsmittel, ein Opiatanalgetikum, ein Geschlechtshormon, ein Stimulanz oder eine therapeutische Mischung davon ist.

17. Orales Wirkstofftransportsystem nach einem der vorherigen Ansprüche, das in einer Einzeldosisform vorliegt.

18. Orales Wirkstofftransportsystem nach Anspruch 17, wobei die Einzeldosisform Kapseln umfasst, die mit dem biliquiden Schaum gefüllt sind.

19. Orales Wirkstofftransportsystem nach Anspruch 18, wobei die Kapseln Hart- oder Weichgelatinekapseln sind.

20. Orales Wirkstofftransportsystem nach einem der Ansprüche 1 bis 16, das in Form eines verdünnbaren Konzentrats vorliegt.

21. Orales Wirkstofftransportsystem nach Anspruch 20, das unendlich in einem Kolösungsmittel verdünnbar ist.

22. Orales Wirkstofftransportsystem nach einem der vorherigen Ansprüche zur Verwendung in einem Verfahren zur Behandlung durch orale Verabreichung am menschlichen oder tierischen Körper.

## Patentansprüche (Patentansprüche für folgende(n) Vertragsstaat(en): DE, GB, FR)

1. Orales Wirkstofftransportsystem, das einen biliquiden Schaum umfasst, umfassend:
1 bis 20 Gewichts-% einer kontinuierlichen hydrophilen Phase,
70 bis 98 Gewichts-% eines pharmazeutisch akzeptablen Öls, das eine diskontinuierliche Phase bildet, wobei das pharmazeutisch akzeptable Öl einen schlecht wasserlöslichen Wirkstoff in einer Menge von 0,1 bis 20 Gewichts-% darin aufgelöst oder dispergiert hat,
wobei der schlecht wasserlösliche Wirkstoff ein Wirkstoff ist, der sich in Wasser in einer Menge von weniger als 1 Gewichts-% auflöst,
und der biliquide Schaum 0,5 bis 5 Gewichts-% eines Tensids darin umfasst, um die Bildung eines stabilen biliquiden Schaums zu ermöglichen, wobei alle Prozentangaben auf das Gesamtgewicht der Formulierung bezogen sind.

2. Orales Wirkstofftransportsystem nach Anspruch 1, wobei die kontinuierliche hydrophile Phase eine wässrige Phase ist.

3. Orales Wirkstofftransportsystem nach Anspruch 2, wobei die wässrige Phase Wasser ist.

4. Orales Wirkstofftransportsystem nach Anspruch 2, wobei die wässrige Phase ein Salz oder ein Kolösungsmittel darin umfasst.

5. Orales Wirkstofftransportsystem nach Anspruch 1, wobei die kontinuierliche hydrophile Phase ein nicht-wässriges Lösungsmittel ist.

6. Orales Wirkstofftransportsystem nach Anspruch 5, wobei das nicht-wässrige Lösungsmittel ein aliphatischerAlkohol, Polyethylenglycol, Propylenglycol oder Glycerol oder Mischungen davon ist.

7. Orales Wirkstofftransportsystem nach einem der vorherigen Ansprüche, wobei das pharmazeutisch akzeptable Öl ein Mono-, Di- oder Triglycerid oder eine Mischung davon ist.

8. Orales Wirkstofftransportsystem nach Anspruch 7, wobei die Mono-, Di- oder Triglyceride die Glycerolester von Fettsäuren sind, die 6 bis 22 Kohlenstoffatome enthalten.

9. Orales Wirkstofftransportsystem nach einem der vorherigen Ansprüche, wobei das Tensid einen Alkylpolyglycolether, einen Alkylpolyglycolester, einen ethoxylierten Alkohol, einen Polyoxyethylensorbitanfettsäureester, einen Polyoxyethylenfettsäureester, einen Polyoxyethylenfettsäureester, ein ionisches oder nicht-ionisches Tensid, ein hydriertes Castoröl/Polyoxyethylenglycoladdukt, das 25 bis 60 Ethoxygruppen enthält, ein Castoröl/Polyoxyethylenglycoladdukt, das 25 bis 45 Ethoxygruppen enthält oder Mischungen davon umfasst.

10. Orales Wirkstofftransportsystem nach einem der vorherigen Ansprüche, das darin einen Co-Emulgator in einer Menge umfasst, die ausreichend ist, um das Löslichmachen des schlecht wasserlöslichen Wirkstoffs zu vollenden.

11. Orales Wirkstofftransportsystem nach Anspruch 10, wobei der Co-Emulgator ein Phosphoglycerid oder ein Phospholipid ist.

12. Orales Wirkstofftransportsystem nach einem der vorherigen Ansprüche, wobei die diskontinuierliche Phase 85 bis 96 Gewichts-% des biliquiden Schaums umfasst.

13. Orales Wirkstofftransportsystem nach Anspruch 12, wobei die diskontinuierliche Phase 90 bis 95 Gewichts-% des biliquiden Schaums umfasst.

14. Orales Wirkstofftransportsystem nach einem der vorherigen Ansprüche, wobei die kontinuierliche hydrophile Phase 2 bis 10 Gewichts-% des biliquiden Schaums umfasst.

15. Orales Wirkstofftransportsystem nach einem der vorherigen Ansprüche, wobei der schlecht wasserlösliche Wirkstoff ein analgetisches oder anti-inflammatorisches Mittel, ein Anthelmintikum, ein anti-arrhythmisches Mittel, ein anti-Koagulanz, ein anti-Deppressivum, ein anti-Diabetikum, ein anti-Epileptikum, ein anti-Pilzmittel, ein anti-Gichtmittel, ein anti-Hypertoniemittel, ein anti-Malaria-, ein anti-Migränemittel, ein anti-Muskarinikum, ein anti-Neoplastikum, ein anti-Protozoenmittel, ein anti-Schilddrüsenmittel, ein Anxiolytikum, Sedativum, hypnotisches oder neuroleptisches Mittel, ein Corticosteroid, in Diuretikum, ein anti-Parkinsonmittel, ein Mittel für den Magen-Darm-Trakt, ein Histamin-H Rezeptor Antagonist, ein Lipid-Regulierungsmittel, ein anti-anginöses Mittel, ein Ernährungsmittel, ein Opiatanalgetikum, ein Geschlechtshormon, ein Stimulanz oder eine therapeutische Mischung davon ist.

16. Orales Wirkstofftransportsystem nach einem der vorherigen Ansprüche, das in einer Einzeldosisform vorliegt.

17. Orales Wirkstofftransportsystem nach Anspruch 16, wobei die Einzeldosisform Kapseln umfasst, die mit dem biliquiden Schaum gefüllt sind.

18. Orales Wirkstofftransportsystem nach Anspruch 17, wobei die Kapseln Hart- oder Weichgelatinekapseln sind.

19. Orales Wirkstofftransportsystem nach einem der Ansprüche 1 bis 15, das in Form eines verdünnbaren Konzentrats vorliegt.

20. Orales Wirkstofftransportsystem nach Anspruch 19, das unendlich in einem Kolösungsmittel verdünnbar ist.

21. Orales Wirkstofftransportsystem nach einem der vorherigen Ansprüche zur Verwendung in einem Verfahren zur Behandlung durch orale Verabreichung am menschlichen oder tierischen Körper.

## Revendications (Revendications pour l'(les) Etat(s) contractant(s) suivant(s): DE, GB, FR)

1. Système d'administration de médicament par voie orale, qui comprend une mousse biliquide comprenant :
1 à 20 % en poids d'une phase hydrophile continue,
70 à 98 % en poids d'une huile pharmaceutiquement acceptable qui forme une phase discontinue, ladite huile pharmaceutiquement acceptable renfermant à l'état dissous ou dispersé un médicament médiocrement hydrosoluble en une quantité de 0,1 à 20 % en poids,
ledit médicament médiocrement hydrosoluble étant un médicament qui se dissout dans l'eau en une quantité inférieure à 1 % en poids,
et la mousse biliquide renfermant 0,5 à 5 % en poids d'un agent tensio-actif pour permettre la formation d'une mousse biliquide stable, tous les pourcentages étant basés sur le poids total de la formulation, sous réserve que le système d'administration du médicament par voie orale ne comprenne pas une mousse biliquide contenant :
**Phase aqueuse**
| | |
|---|---|
| Eau | 9,9 % |
| Tween 20 (Polysorbate 20) | 0,1 % |
**Phase huileuse**
| | |
|---|---|
| Ibuprofène | 4,5 % |
| Myristate d'isopropyle | 84,5 % |
| Lawreth 3 | 1 % |

2. Système d'administration de médicament par voie orale suivant la revendication 1, dans lequel la phase hydrophile continue est une phase aqueuse.

3. Système d'administration de médicament par voie orale suivant la revendication 2, dans lequel la phase aqueuse est l'eau.

4. Système d'administration de médicament par voie orale suivant la revendication 2, dans lequel la phase aqueuse renferme un sel ou un co-solvant.

5. Système d'administration de médicament par voie orale suivant la revendication 1, dans lequel la phase hydrophile continue est un solvant non aqueux.

6. Système d'administration de médicament par voie orale suivant la revendication 5, dans lequel le solvant non aqueux est un alcool aliphatique, le polyéthylène-glycol, le propylèneglycol ou le glycérol, ou bien leurs mélanges.

7. Système d'administration de médicament par voie orale suivant l'une quelconque des revendications précédentes, dans lequel l'huile pharmaceutiquement acceptable est un mono-, di- ou triglycéride ou un de leurs mélanges.

8. Système d'administration de médicament par voie orale suivant la revendication 7, dans lequel les mono-, di- ou triglycérides sont les esters de glycérol d'acides gras contenant 6 à 22 atomes de carbone.

9. Système d'administration de médicament par voie orale suivant l'une quelconque des revendications précédentes, dans lequel l'agent tensio-actif comprend un éther alkylique de polyglycol, un ester alkylique de polyglycol, un alcool éthoxylé, un ester d'acide gras de polyoxyéthylène-sorbitanne, un ester d'acide gras de polyoxyéthylène, un ester d'acide gras de polyoxyéthylène, un agent tensio-actif ionique ou non ionique, un produit d'addition huile de ricin hydrogénée/polyoxyéthylèneglycol contenant 25 à 60 groupes éthoxy, un produit d'addition huile de ricin/polyoxyéthylèneglycol contenant 25 à 45 groupes éthoxy, ou leurs mélanges.

10. Système d'administration de médicament par voie orale suivant l'une quelconque des revendications précédentes, qui renferme un co-émulsionnant en une quantité suffisante pour achever la solubilisation du médicament médiocrement hydrosoluble.

11. Système d'administration de médicament par voie orale suivant la revendication 10, dans lequel le co-émulsionnant est un phosphoglycéride ou un phospholipide.

12. Système d'administration de médicament par voie orale suivant l'une quelconque des revendications précédentes, dans lequel la phase discontinue représente 85 à 96 % en poids de la mousse biliquide.

13. Système d'administration de médicament par voie orale suivant la revendication 12, dans lequel la phase discontinue représente 90 à 95 % en poids de la mousse biliquide.

14. Système d'administration de médicament par voie orale suivant l'une quelconque des revendications précédentes, dans lequel la phase hydrophile continue représente 2 à 10 % en poids de la mousse biliquide.

15. Système d'administration de médicament par voie orale suivant l'une quelconque des revendications précédentes, dans lequel le médicament médiocrement hydrosoluble est un analgésique ou un agent anti-inflammatoire, un anthelmintique, un agent anti-arythmique, un agent anticoagulant, un antidépresseur, un antidiabétique, un anti-épileptique, un agent antifongique, un agent antigoutteux, un agent anti-hypertension, un agent antipaludique, un agent antimigraineux, un agent antimuscarinique, un agent antinéoplasique, un agent antiprotozoaires, un agent antithyroïdien, un anxiolytique, un sédatif, un hypnotique ou un agent neuroleptique, un corticostéroïde, un diurétique, un anti-Parkinsonien, un agent gastro-intestinal, un antagoniste du récepteur d'histamine H, un agent régulant les taux de lipides, un agent anti-angoreux, un agent nutritionnel, un analgésique opioïde, une hormone sexuelle, un stimulant ou un de leurs mélanges thérapeutiques.

16. Système d'administration de médicament par voie orale suivant la revendication 15, dans lequel le médicament médiocrement hydrosoluble est un anthelmintique, un agent anti-arythmique, un agent anticoagulant, un antidépresseur, un antidiabétique, un anti-épileptique, un agent antifongique, un agent antigoutteux, un agent anti-hypertension, un agent antipaludique, un agent antimigraineux, un agent antimuscarinique, un agent antinéoplasique, un agent antiprotozoaires, un agent antithyroïdien, un anxiolytique, un sédatif, un hypnotique ou un agent neuroleptique, un corticostéroïde, un diurétique, un anti-Parkinsonien, un agent gastro-intestinal, un antagoniste du récepteur d'histamine H, un agent régulant les taux de lipides, un agent anti-angoreux, un agent nutritionnel, un analgésique opioïde, une hormone sexuelle, un stimulant ou un de leurs mélanges thérapeutiques.

17. Système d'administration de médicament par voie orale suivant l'une quelconque des revendications précédentes, qui est sous une forme posologique unitaire.

18. Système d'administration de médicament par voie orale suivant la revendication 17, dans lequel la forme posologique unitaire comprend des capsules remplies de la mousse biliquide.

19. Système d'administration de médicament par voie orale suivant la revendication 18, dans lequel les capsules sont des gélules ou des capsules de gélatine molle.

20. Système d'administration de médicament par voie orale suivant l'une quelconque des revendications 1 à 16, qui est sous forme d'un concentré pouvant être dilué.

21. Système d'administration de médicament par voie orale suivant la revendication 20, qui peut être dilué à l'infini dans un co-solvant.

22. Système d'administration de médicament par voie orale suivant l'une quelconque des revendications précédentes, destiné à être utilisé dans une méthode de traitement par administration orale à l'organisme d'un être humain ou d'un animal.

## Revendications (Revendications pour l'(les) Etat(s) contractant(s) suivant(s): GB, DE, FR)

1. Système d'administration de médicament par voie orale, qui comprend une mousse biliquide comprenant :
1 à 20 % en poids d'une phase hydrophile continue,
70 à 98 % en poids d'une huile pharmaceutiquement acceptable qui forme une phase discontinue, ladite huile pharmaceutiquement acceptable renfermant à l'état dissous ou dispersé un médicament médiocrement hydrosoluble en une quantité de 0,1 à 20 % en poids,
ledit médicament médiocrement hydrosoluble étant un médicament qui se dissout dans l'eau en une quantité inférieure à 1 % en poids,
et la mousse biliquide renfermant 0,5 à 5 % en poids d'un agent tensio-actif pour permettre la formation d'une mousse biliquide stable, tous les pourcentages étant basés sur le poids total de la formulation.

2. Système d'administration de médicament par voie orale suivant la revendication 1, dans lequel la phase hydrophile continue est une phase aqueuse.

3. Système d'administration de médicament par voie orale suivant la revendication 2, dans lequel la phase aqueuse est l'eau.

4. Système d'administration de médicament par voie orale suivant la revendication 2, dans lequel la phase aqueuse renferme un sel ou un co-solvant.

5. Système d'administration de médicament par voie orale suivant la revendication 1, dans lequel la phase hydrophile continue est un solvant non aqueux.

6. Système d'administration de médicament par voie orale suivant la revendication 5, dans lequel le solvant non aqueux est un alcool aliphatique, le polyéthylène-glycol, le propylèneglycol ou le glycérol, ou bien leurs mélanges.

7. Système d'administration de médicament par voie orale suivant l'une quelconque des revendications précédentes, dans lequel l'huile pharmaceutiquement acceptable est un mono-, di- ou triglycéride ou un de leurs mélanges.

8. Système d'administration de médicament par voie orale suivant la revendication 7, dans lequel les mono-, di- ou triglycérides sont les esters de glycérol d'acides gras contenant 6 à 22 atomes de carbone.

9. Système d'administration de médicament par voie orale suivant l'une quelconque des revendications précédentes, dans lequel l'agent tensio-actif comprend un éther alkylique de polyglycol, un ester alkylique de polyglycol, un alcool éthoxylé, un ester d'acide gras de polyoxyéthylène-sorbitanne, un ester d'acide gras de polyoxyéthylène, un ester d'acide gras de polyoxyéthylène, un agent tensio-actif ionique ou non ionique, un produit d'addition huile de ricin hydrogénée/polyoxyéthylèneglycol contenant 25 à 60 groupes éthoxy, un produit d'addition huile de ricin/polyoxyéthylèneglycol contenant 25 à 45 groupes éthoxy, ou leurs mélanges.

10. Système d'administration de médicament par voie orale suivant l'une quelconque des revendications précédentes, qui renferme un co-émulsionnant en une quantité suffisante pour achever la solubilisation du médicament médiocrement hydrosoluble.

11. Système d'administration de médicament par voie orale suivant la revendication 10, dans lequel le co-émulsionnant est un phosphoglycéride ou un phospholipide.

12. Système d'administration de médicament par voie orale suivant l'une quelconque des revendications précédentes, dans lequel la phase discontinue représente 85 à 96 % en poids de la mousse biliquide.

13. Système d'administration de médicament par voie orale suivant la revendication 12, dans lequel la phase discontinue représente 90 à 95 % en poids de la mousse biliquide.

14. Système d'administration de médicament par voie orale suivant l'une quelconque des revendications précédentes, dans lequel la phase hydrophile continue représente 2 à 10 % en poids de la mousse biliquide.

15. Système d'administration de médicament par voie orale suivant l'une quelconque des revendications précédentes, dans lequel le médicament médiocrement hydrosoluble est un analgésique ou un agent anti-inflammatoire, un anthelmintique, un agent anti-arythmique, un anticoagulant, un antidépresseur, un antidiabétique, un anti-épileptique, un agent antifongique, un agent antigoutteux, un agent anti-hypertension, un antipaludique, un agent antimigraineux, un agent antimuscarinique, un agent antinéoplasique, un agent antiprotozoaires, un agent antithyroïdien, un anxiolytique, un sédatif, un hypnotique ou un agent neuroleptique, un corticostéroïde, un diurétique, un agent anti-Parkinsonien, un agent gastro-intestinal, un antagoniste du récepteur d'histamine H, un agent régulant les taux de lipides, un agent anti-angoreux, un agent nutritionnel, un analgésique opioïde, une hormone sexuelle, un stimulant ou un de leurs mélanges thérapeutiques.

16. Système d'administration de médicament par voie orale suivant l'une quelconque des revendications précédentes, qui est sous une forme posologique unitaire.

17. Système d'administration de médicament par voie orale suivant la revendication 16, dans lequel la forme posologique unitaire comprend des capsules remplies de mousse biliquide.

18. Système d'administration de médicament par voie orale suivant la revendication 17, dans lequel les capsules sont des gélules ou des capsules de gélatine molle.

19. Système d'administration de médicament par voie orale suivant l'une quelconque des revendications 1 à 15, qui est sous forme d'un concentré pouvant être dilué.

20. Système d'administration de médicament par voie orale suivant la revendication 19, qui peut être dilué à l'infini dans un co-solvant.

21. Système d'administration de médicament par voie orale suivant l'une quelconque des revendications précédentes, destiné à être utilisé dans une méthode de traitement par administration orale à l'organisme d'un être humain ou d'un animal.
